# EUROPEAN PATENT APPLICATION

(11) **EP 2 601 938 A1**
(43) Date of publication of application: **12.06.2013**
(21) Application number: 12008098.1
(22) Date of filing: 04.12.2012
(51) Int. Cl.: A61K 9/24, A61K 31/315, A61K 31/4164, A61K 31/43, A61K 31/4439, A61K 31/549, A61K 31/7048, A61K 45/06, A61P 1/04, A61P 31/04

(54) **Pharmaceutical compositions for the treatment of Helicobacter pylori associated diseases**

(30) Priority: 05.12.2011 EP 11192001
(71) Applicant: Al-Mehdar, Abo Bakr Mohammed Ali, Riyadh (SA)
(72) Inventor: Al-Mehdar, Abo Bakr Mohammed Ali, Riyadh (SA)
(74) Representative: Lins, Martina

(57) **Abstract**

An oral pharmaceutical composition for the treatment of helicobacter pylori associated diseases is disclosed comprising a proton pump inhibitor, an antibiotic or a combination of antibiotics, and additionally at least one agent selected from the group consisting of a) taurolidine or taurultam or a combination thereof, b) a zinc amino acid chelate, c) tromethamole, within a fixed-dose combination for oral administration in the form of either an instant release formulation, for example a compressed tablet, granules or the like, or in the form of a biphasic release form, for example a tablet with a slow release core and a compressed outer fast release layer, wherein the tromethamole is always present in instant release formulations.

## Description

The invention is related to a pharmaceutical composition for the treatment of helicobacter pylori associated diseases like ulcers and cancer, which is a composition for oral administration and comprises an acid blocker like a proton pump inhibitor together with at least one antibiotic.

The relationship between gastrointestinal ulcers and infection with Helicobacter pylori proposed in 1983 by Warren (Warren j. R., Lancet 1983; 1., 1273) is well established. Moreover duodenal ulcers and certain types of cancer are also associated with Helicobacter infections. A number of different therapies have been proposed for treatment of H. pylori infections. Most of these therapies comprise different combinations of antibacterial compounds. Some of the combination therapies comprise a proton pump inhibitor and one or more antibacterial compounds, for instance a combined regimen of omeprazole and amoxicillin which has been approved by regulatory authorities in for example Great Britain and Sweden for the treatment of H. pylori infections. Different triple therapies, for example omeprazole, clarithromycin and amoxicillin or other antibacterial substances, have been reported.

WO 93/00327, Astra Aktiebolag, discloses the combination of a substance with inhibiting effect on the gastritic acid secretion which increases the intra-gastric pH and an antibacterial compound.

WO 92/03135, Smithkline & French Laboratories, discloses a combination of a benzimidazole and an anti-Helicobacter agent, i.e. for instance pantoprazole in combination with amoxicillin and/or metronidazole.

Initially, treatment was based on administering antacids, such as magnesium or aluminium compounds, calcium carbonates, alkaline bismuth salts, e.g. bismuth aluminates, colloidal bismuth salts. A high relapse rate of over 80 % and side effects, such as the rebound effect of acid secretion, deposits of aluminium and bismuth salts in the tissue to bismuth nephropathy, and bismuth encephalopathy forced the medical field to pursue new paths.

After proof has been secured of H. pylori following gastroscopy and removal of the mucosa of the stomach from the antrum or corpus, eradication of the germ through combination therapy is necessary. Serious H. pylori related forms of gastritis and underdevelopment should be treated in accordance with cancer prophylaxis to stomach carcinoma and lymphoma (MALT). Treatment turned out to be very difficult. Due to a high relapse rate, the mono- and dual-therapy were insufficient. For mono-therapy with bismuth compounds, and for dual-therapy with amoxicillin and omeprazole, the eradication rate was only between 35 and 60 %. Through the combination of bismuth, amoxicillin and metronidazole, healing was achieved for the first time in 85 - 95 % of the cases after a 4-week treatment. The toxicity of this 3-fold combination, however, has not yet been clarified, especially when taking into account the long treatment time of 4 weeks.

Proton pump inhibitors are susceptible to degradation or transformation in acid reacting and neutral media. In respect to stability, it is obvious that the proton pump inhibitor as an active ingredient should be protected from contact with acidic gastric juice, for example by an enteric coating layer. There are different enteric coating layered preparations of omeprazole as well as other proton pump inhibitors described in the prior art, see for example US 4 786 505 B (Hassle).

WO 96/24357 discloses a combination of an acid susceptible proton pump inhibitor and one or more antibacterial compounds in a fixed formulation intended for oral use and in the form of an enteric coating layered tablet, a capsule or a multiple unit tableted dosage form. Preferably the proton pump inhibitor is prepared in the form of individually enteric coating layered pellets and the pellets are filled into a capsule together with the antibacterial compounds optionally mixed with pharmaceutically acceptable excipients. It has been found that the pellet cover layer might be damaged during the manufacture of the tablets.

US 2004/0082514 A1 discloses oral administration of pentagastrin in conjunction with a gastric proton pump inhibitor, i.a. for the treatment of pathological conditions associated with Helicobacter infections. The pentagastrin exerts a local effect in the stomach and shows in combination with a proton pump inhibitor a synergistic effect in eradicating H. pylori. Organic acids are preferred as pH modifying agents within therapeutical compositions. These compositions are not suited for patients with sensitive stomach or patients who are sensitive to acids.

Still most favoured is a triple therapy with a proton pump inhibitor (abbreviated as "PPI") and two antibiotics. The triple therapy is a 7-day treatment. According to the "Italian Triple Therapy" the antibiotics are clarithromycin and metronidazole and according to the "French Triple Therapy" these are clarithromycin and amoxicillin. The medication must be taken twice daily each in standard dosage. This means that the patient must take many tablets a day. Also, there are side effects of the antibiotics, especially diarrhoea, taste changes etc. An antibiotics resistance development is also disadvantageous. H. pylori resistance to metronidazole, clarithromycin and amoxicillin has already been found, in the dual, triple and sequential therapy that are proposed to treat gastric ulcer associated with H. pylori infection, each singe active substance is administered separately in different dosage forms each of them comprises only one single active substance.

It is well known that patient compliance is a main factor in receiving a good result in medical treatments especially in the treatment of H. pylori infections. It is therefore an objective of the present invention to propose an oral formulation for treatment of helicobacter pylori associated diseases comprising an acid blocker in the form of a proton pump inhibitor together with at least one antibiotic, which has a better patient compliance. According to further aspects of the invention the drawbacks in the state of the art should be reduced, side effects should be reduced, eradication rate improved, and acceptance ameliorated.

The objects of invention are achieved by the features of claim 1 as attached. The oral pharmaceutical composition or the orally administered formulation of the invention, resp., for the treatment of helicobacter pylori associated diseases comprises
- a proton pump inhibitor, preferably omeprazole,
- an antibiotic or a combination of antibiotics, that is so far a dual or triple therapy as known from the state of the art, and according to the invention combined with
- additionally at least one agent selected from the group consisting of a) taurolidine or taurultam or a combination thereof, b) a zinc amino acid chelate, c) tromethamole (TRIS),
within a fixed-dose combination in the form of either an instant release formulation, for example a compressed tablet, granules or the like, or in the form of a biphasic release form, for example a tablet with a slow release core and a compressed outer fast release layer, wherein the tromethamole is always present in any instant release formulations according to this invention.

The formulation optionally comprises suitable, pharmaceutical acceptable excipients.

According to a first aspect of the invention it is especially preferred that the composition according to the invention comprises, additionally to the antibiotics and the proton pump inhibitor, taurolidine, taurultam or a combination thereof. This is for the following reasons.

The fixed-dose formulation according to the invention comprises at least one conventional antibiotic. However H. pylori is often resistant to a wide variety of conventionally used antibiotics including amoxicillin, clarithromycin, metronidazole, tinidazole and a wide range of other antibiotics. It has been reported that cure rate is reduced from over 90 % to less than 60 % with increasing incidence of infection relapse. Therefore, an urgent task to be solved is reducing bacterial resistance and improving cure rates. To provide an enhanced Helicobacter pylori eradication, one or more conventional antibiotics are combined with an amount of an antimicrobial medicament selected from the group consisting of taurolidine, taurultam or a combination thereof.

Taurolidine ([bis(1,1-dioxoperhydro-1,2,4-thiadiazinyl-4)]-methane) is a drug with anti-infective, antimicrobial and anti-lipopolysaccharide properties. The WHO has classified taurolidine with ATC code B05CA05 as an anti-infective usually used in irrigating solutions. It is supposed to have anti-mycotic effects and to neutralize endotoxines as well. Derived from the amino acid taurine, its immune modulatory action is reported to be mediated with priming and activation of macrophages and polymorphonuclear leukocytes. Taurolidine has been used to treat patients with peritonitis and as an agent in patients with systemic inflammatory response syndrome. Additionally, taurolidine demonstrates some anti-tumour properties, with positive results seen in early-stage clinical investigations using the drug to treat gastrointestinal malignancies and tumours of the central nervous system. T

It has now been found that taurolidine and/or taurultam reduce significantly H. pylori resistance to conventional antibiotics and reduce the risk for developing bacterial resistance. Antibiotics and taurolidine/taurultam produce synergistic effects in eradication and cure of H. pylori associated diseases, including cancer. Taurolidine and taurultam are cell-wall cross-linking compounds effective in the treatment of gastric and duodenal ulcer initiated by H. pylori. Taurolidine has an exceptionally broad spectrum of antimicrobial and antibacterial activity including activity against gram positive and gram negative, aerobic, and anaerobic bacteria. Resistance has not been observed either in vivo or in vitro. The compounds taurolidine and taurultam are disclosed in US 5 210 083. The mechanism of taurolidine action is unlike that of antibiotics and is based on a chemical reaction. While not being bound by any theory, during the metabolism of taurolidine to taurinamide and ultimately taurine and water, methylol groups are liberated which chemically react with the mureins in the bacterial cell walls, and this results in the denaturing of the complex polysaccharide and liposaccharide components of the bacterial cell wall as well as changing the double stranded DNA of the plasmid to a denatured or single stranded DNA. Taurolidine has been shown to be safe and well tolerated at systemic doses exceeding 40 g/day and cumulative doses up to and exceeding 300 g. In the combined therapy with antibiotics taurolidine and/or taurultam prevent resistances to the antibiotics that could finally lead to multi-resistant H. pylori strains. The supposed anti-neoplasia effect of taurolidine/taurultam creates a cancer prophylactic effect during H. pylori therapy and eradication. Together antibiotics and taurolidine/taurultam are fast and highly effective thereby shortening the duration of treatment considerably. Taurolidine seems to be more effective than taurultam, and its presence in the composition is therefore especially preferred. Nevertheless, whenever taurolidine is mentioned in this description of the invention it could be replaced by taurultam or a combination of both.

According to a second aspect of the invention, the fixed-dose formulation may also comprise a zinc amino acid chelate for strengthening the stomach mucosa, sticking to the stomach wall and acting as a buffer to gastric acid, serving as an antioxidant, controlling the inflammatory response to stomach injury, and inhibiting the growth of H. pylori bacteria probably by strengthening the stomach mucosa and making it less susceptible to a bacterial infection. This can be an additional measure or a measure per se. Within the composition the zinc amino acid chelate is a separate curative agent, it prevents acidification, protects the stomach mucosa and thereby slows down growth of the bacteria. The zinc amino acid chelate thus has an effect of its own but also synergetically assists the antibiotic treatment with or without taurolidine.

It is therefore most preferred when group a) substances (taurolidine group, as cited above) and a zinc amino acid chelate are both present in the composition according to this invention.

Generally the fixed-dose pharmaceutical composition according to the invention may comprise any useful antibiotics, especially nitroiridazole, tetracycline, penicillins, cephalosporins, carbopenems, amino glycosides, macrolide antibiotics, lincosamide antibiotics, 4-quinolones, rifamycins and nitrofurantoin.

Examples of such antibiotics and antibacterial compounds are: ampicillin, amoxicillin, benzyl penicillin, phenoxymethylpenicillin, bacampicillin, picampicillin, carbenicillin, cloxacillin, cyclacillin, dicloxacillin, methicillin, oxacillin, piperacillin, ticarcillin, flucloxacillin, cefuroxime, cefetamet, cefetrame, cefixime, cefoxitin, ceftazidime, ceftizoxime, latamoxef, cefoperazone, ceftriaxone, cefsu-Iodin, cefotaxime, cephalexin, cefaclor, cefadroxil, cefalothin, cefazolin, cefpodoxime, ceftibuten, aztreonam, tigemonam, erythromycin, dirithromycin, roxithromycin, azithromycin, clarithromycin, clindamycin, paldimycin, lincomycin, vancomycin, spedtinomycin, tobramycin, paromomycin, metronidazole, tinidazole, ornidazole, amifloxacin, conoxacin, ciprovloxacin, difloxacin, enoxacin, fleroxacin, norfloxacin, ofloxacin, temafloxacin, doxycycline, minocycline, tetracycline, chlortetracycline, oxytetracycline, methacycline, rloitetracyclin, nitrofurantoin, nalidixic acid, gentamycin, rifampicin, amikacin, netilmicin, imipenem, cilastatin, chloramphenicol, furazolidone, nifuroxyzide, sulfadiazine, sulfametoxazol, bismuth subsalicylate, colloidal bismuth subcitrate, gramicidin, mecillinam, cloxiquine, chlorhexidine, dichlorobenzylalcohol, methyl-2-pentylphenol.

The active antibacterial agents could be in standard forms or used as salts, hydrates, esters etc. However, amoxicillin or a combination of tinidazole and clarithromycin is preferred.

The proton pump inhibitor may be used in neutral form or in the form of an alkaline salt, such as for instance the Mg²⁺, Ca²⁺, Na.sup., K.sup. or Li.sup. The compounds may be used in racemic form or in the form of a substantially pure enantiomer thereof, or alkaline salts of the single enantiomer. Suitable proton pump inhibitors are for example disclosed in EP 0005129 A1, EP 174 726 A1, EP 166 287 A1, GB 2 163 747 A and WO 90/06925, WO 91/19711, WO 91/19712, and further especially suitable compounds are described in WO 95/01977 and WO 94/27988. Omeprazole is preferred. Omeprazole can be replaced by Pantoprazole.

According to another aspect of the invention, the composition additionally comprises tromethamole. Thromethamole (INN) also known as tri(hydroxymethyl)-aminomethane, 2-Amino-2-(hydroxymethyl)-propan-1,3-diol, TRIS, THAM, tromethamin (CAS 77-86-1), and it is widely used in biochemistry as a component of buffer solutions. The buffering capacity of TRIS buffer lies in the range of pH above 7 to 9. The tromethamole stabilizes the proton pump inhibitor and other combined active ingredients against gastric acid degradation. It raises the stomach pH above the pKa of the acid susceptible PPIs and hence increases their resistance against gastric acid degradation. Tromethamole can be incorporated in considerably small quantities in the formulation. It has been reported to prepare PPIs, e.g. omeprazole in the form of immediate release dosage form in combination with sodium bicarbonate and/or Magnesium hydroxide. Such alkalinizers have to be incorporated in considerably large amounts which are quite bulky to fit in multi component fixed dosage forms. In addition to that cautions are to be considered when giving sodium salts to hypertensive patients. Tromethamole buffers gastric acid successfully in a much smaller dose ranging between 50 to 300 mg per dose.

By the use of tromethamole new instant release formulations are available which are superior to previously known ones. It is thus possible to have Tromethamole as the sole additional agent in combination with the PPI and the antibiotic(s) within an instant release formulation or within a biphasic release form, preferably within the fast release layer or phase, and in preferred embodiments in the outer layer of a biphasic tablet where the PPI is in the core slow release layer/phase, e.g. with an enteric coating. The tromethamole protects the intestine and stomach against acid attack and buffers the PPI in case the enteric cover is damaged or not a perfect cover.

In the preferred embodiments the tromethamole is present in the composition in combination with either at least one agent from the taurolidine-group, preferably with taurolidine, or with a zinc amino acid chelate. It is most preferred that all three agents, i.e. taurolidine, zinc amino acid chelate and tromethamole, are comprised in the composition according to the invention.

Independent of the type of formulation it is preferred that the antibiotic is amoxicillin or a combination of tinidazole and clarithromycin.

Independent of the type of formulation it is preferred that the PPI is omeprazole.

The pharmaceutical composition according to the invention may be in the form of capsules, effervescent granules or powder, effervescent tablets, liquids, dispersible and/or soluble tablets, dispersible powder, suspensions, sachets, pre-mix syrups, jellies, glycerites or tablets. Preferred are compressed tablets. Dispersible formulations are suitable for children and patients with swallowing disorders. Dispersible powders and tablets are also suitable for naso-gastric administration.

The fixed-dose composition according to the invention may be an instant release formulation. This avoids known problems with fixed unit dosage forms comprising rather high amount of active substances. It is well known that preparation of fixed unit tableted dosage form raises specific problems when enteric coating layered pellets containing acid susceptible proton pump inhibitors as active substance are compressed into tablets. Since the enteric coating layer often does not withstand the compression of the pellets into tablets, the susceptible active substance will be destroyed upon administration by penetrating acidic gastric juice and the acid resistance of the enteric coating layer of the pellets will not be sufficient in the tablet after compression. The instant release formulation releases their active ingredients in the stomach for immediate effects and is devoid of the lengthy enteric coating procedures that produces dosage forms with delayed action. All the instant release formulations according to the invention comprise tromethamole.

According to a further aspect of the invention, the fixed-dose composition may be formulated as a biphasic release form. Preferably as a biphasic tablet with an inner slow release layer and an outer fast release layer (core and shell).

The biphasic release of the ingredients can be achieved, if the inner layer is an enteric coated core. Especially proton pump inhibitors like omeprazole are acid-unstable. The enteric coating prevents release of the active ingredient or the active ingredients inside the core before they reach the small intestine. The delayed release avoids acid exposure to the stomach and delivers them to the basic pH environment of the intestine. Thus it is preferred that the PPI is comprised within the inner layer, the slow release phase or the core, respectively. The core, or inner layer, is coated by compression over the whole surface with a fast-disintegrating layer, preferably formulated as a compressed tablet layer or shell. The outer layer, respectively the compressed tablet, comprises the antibiotic or the combination of antibiotics.

It is preferred that the outer layer or shell also comprises taurolidine or at least one member of the taurolidine group a).

It is also preferred that the outer layer or shell also comprises the tromethamole.

It is further preferred that the outer layer or shell comprises the zinc amino acid chelate.

It is further preferred that the slow release layer or core comprises an amount of the antibiotic or an amount of the combined antibiotics and the fast release layer comprises the remaining amount of the antibiotic or the remaining amount of the combined antibiotics.

Thus there are the following most preferred embodiments for a biphasic release form:

| **Core/inner layer/slow release phase** | **Shell/outer layer/fast release phase** |
|---|---|
| PPI, (optionally additives/supplies/excipients) | Antibiotic(s) (optionally additives..), taurolidine |
| PPI, (optionally additives/supplies/excipients) | Antibiotic(s) (optionally additives..), zinc amino acid chelate |
| PPI, (optionally additives/supplies/excipients) | Antibiotic(s) (optionally additives..), taurolidine and zinc amino acid chelate |
| PPI, (optionally additives/supplies/excipients) | Antibiotic(s) (optionally additives..), taurolidine and tromethamole |
| PPI (optionally additives/supplies/excipients) | Antibiotic(s) (optionally additives..), zinc amino acid chelate and tromethamol |
| PPI, (optionally additives/supplies/excipients) | Antibiotic(s) (optionally additives..), taurolidine, tromethamole, zinc amino acid chelate |

In all of the above cited embodiments part of the antibiotic(s) can be added to the core phase.

There are the following preferred embodiments for an instant release formulation (in the form of tablets, powder, sachets, granules, most preferred compressed tablets):

| |
|---|
| PPI: antibiotic(s), taurolidine, tromethamole, additives/excipients |
| PPI: antibiotic(s), zinc amino acid chelate, tromethamole, additives/excipients |
| PPI: antibiotic(s), taurolidine, zinc amino acid chelate, tromethamole, additives/excipients |

According to a further aspect of the invention there is disclosed a method for treatment of helicobacter pylori associated diseases comprising administering orally to a patient in need thereof, who is a human being or in general a mammal,
- a proton pump inhibitor,
- an antibiotic or a combination of antibiotics,
- additionally at least one agent selected from the group consisting of taurolidine or taurultam or a combination thereof, a zinc amino acid chelate, tromethamole,
where said components are arranged within one or more pharmaceutical composition(s) administered concomitantly or sequentially over a time period from minutes to hours.

This means that the pharmaceutical compositions according to the invention, as described previously, could also be separated/distributed in two or several compositions for concomitant administration without departure from the spirit of invention. A sequential administration of the ingredients is possible as well. The administration could then mimic a biphasic release composition. For example part of the antibiotics or the complete dose of antibiotics could be administered first and preferably together with Taurolidine, tromethamole and/or zinc amino acid chelate (one of these or all possible combinations of these three optional ingredients), and optionally a second portion of the antibiotics could be given delayed in time with respect to the first dose together with the PPI. Other regimen and schedules for separate administration of the components of the pharmaceutical composition of the invention are possible.

### Detailed description of embodiments:

(1) compressed tablets are made from omeprazole in a dose from 10 to 60 mg, preferably from 20 to 40 mg, amoxicillin in a dose from 500 to 1000 mg, preferably from 500 to 800 mg or from 800 to 1000 mg. The tablets also contain tromethamole used as a buffering alkalinizer to enhance the stability of omeprazole in gastric acidity in a dose from 50 to 300 mg, preferably from 50 to 200 mg, more preferred from 70 to 200 mg. Further a variety of pharmaceutical excipients including starch glycolate at between 50 to 135 mg, Talc at between 50 to 100 mg, Aerosil ® 200 or RTM between 1,5 to 4,5 mg, sodium lauryl sulphate between 1 to 4 mg, sodium stearyl fumarate between 10 to 20 mg, magnesium stearate between 4,5 to 13,5 mg, in addition to other pharmaceutical additives, as far as required and desired.
(2) As in (1), wherein the amoxicillin is substituted with a combination containing both tinidazole and clarithromycin with the rest of ingredients unchanged. Tinidazole is included in the formulation at a dose of between 100 - 500 mg, preferably 250 - 500 mg, clarithromycin is included at a dose between 200 - 500 mg, preferably 250 - 500 mg, more preferably 250 - 400 mg.
   The compressed tablets from embodiment (1) and embodiment (2) are indicated in sequential therapy program for the treatment of gastric and duodenal ulcers associated with H. Pylori infections. The tablets from embodiment (1) are to be administered twice daily for 5 days, followed by the compressed tablets from embodiment (2) to be taken as one tablet twice daily for 5 more consecutive days. The medical practitioner will decide to make use of embodiments (1) and (2) either separately or in combination.
(3) According to embodiment (3) of the invention compressed tablets are made from omeprazole in a dose from 10 to 60 mg, preferably from 20 to 40 mg and more preferably from 20 to 30 mg, amoxicillin at a dose from 500 to 1000 mg, preferably from 500 to 800 or 800 to 1000 mg. The tablet also contains tromethamole as in embodiment (1). Additionally taurolidine/taurultam is included in the formula in order to synergies the activity of the amoxicillin and to reduce bacterial resistance due to its bacterial cell cross-linking effect. Taurolidine/taurultam is combined in a dose between 200 - 400 mg; pharmaceutical excipients may also be present as described in connection with embodiment (1).
(4) According to another embodiment of the invention the amoxicillin in embodiment (3) is substituted with a combination containing both tinidazole and clarithromycin with the rest of the active ingredients in embodiment (3) remaining unchanged. The taurolidine is included in the formula to reduce bacterial resistance against clarithromycin and tinidazole in doses between 100 - 500 mg, preferably either between 100 - 200 mg or between 200 - 400 mg, and more preferably at between 250 - 500 mg. Tinidazole is included in the formulation at a dose of between 100 - 500 mg, preferably between 100 - 200 mg or 300 - 400 mg, and more preferably between 250 - 500 mg. Clarithromycin is included at a dose between 200 - 500 mg, preferably between 200 - 300 mg or 250 - 400 mg, and more preferably from 250 - 500 mg.
   The compressed tablets from embodiment (3) and embodiment (4) are indicated in sequential therapy program for the treatment of gastric and duodenal ulcers associated with H. Pylori infections. The tablets from embodiment (3) are to be administered twice daily for 5 days, followed by the compressed tablets from embodiment (4) to be taken as one tablet twice daily for 5 more consecutive days. The medical practitioner will decide to make use of embodiments (3) and (4) either separately or in combination.
(5) In embodiment (5) the taurolidine included in embodiment (3) is substituted with zinc amino acid chelate in a dose between 10 mg to 100 mg/tablet, preferably between 10 to 20 mg or 25 to 30 mg and most preferably between 40 to 100 mg, with the rest of other active and inactive ingredients (omeprazole, amoxicillin, tromethamole and excipients) remained unchanged. The prepared tablets are preferably film coated.
(6) In embodiment (6) the taurolidine included in embodiment (4) is substituted with zinc amino acid chelate in a dose between 10 mg to 100 mg/tablet, preferably between 10 to 20 mg or 25 to 30 mg and most preferably between 40 to 100 mg, with the rest of other active and inactive ingredients (omeprazole, tinidazole plus clarithromycin, tromethamole and excipients) remained unchanged. The prepared tablets are preferably film coated.
   The compressed tablets from embodiment (5) and embodiment (6) are indicated in sequential therapy program for the treatment of gastric and duodenal ulcers associated with H. Pylori infections. The tablets from embodiment (5) are to be administered twice daily for 5 days, followed by the compressed tablets from embodiment (6) to be taken as one tablet twice daily for 5 more consecutive days. The medical practitioner will decide to make use of embodiments (5) and (6) either separately or in combination.
(7) The embodiment is a combination of embodiments (5) and (3) in that both taurolidine and zinc amino acid chelate are present within this formulation for a compressed tablet. The active and inactive ingredients thus are: omeprazole, amoxicillin, taurolidine, zinc amino acid chelate, tromethamole and excipients. The dose ranges are as in embodiments (3) and (5), respectively.
(8) The embodiment is a combination of embodiments (6) and (4) in that both taurolidine and zinc amino acid chelate are present within this formulation for a compressed tablet. The active and inactive ingredients thus are: omeprazole, clarithromycin poul tinidazole, taurolidine, zinc amino acid chelate, tromethamole and excipients. The dose ranges are as in embodiments (3) and (5), respectively.
(9) According to another embodiment the antibiotic amoxicillin is combined with another antibiotic or antibiotic mixture, preferably in a dose range of 100 - 1000 mg, more preferably 200 - 500 mg of that second antibiotic or mixture of antibiotics, irrespective of the amoxicillin dose which remains unchanged. The second antibiotic can preferably be levofloxacin in a dose range between 200 and 500 mg.
(10) According to another embodiment the antibiotic combination clarithromycin plus tinidazole is combined with a further antibiotic or antibiotic mixture, preferably in a dose range of 100 - 1000 mg, more preferably 200 - 500 mg of that second antibiotic or mixture of antibiotics, irrespective of the clarithromycin and tinidazole doses which remain unchanged. The second antibiotic can preferably be levofloxacin in a dose range between 200 and 500 mg.
(11) In further embodiments according to the invention the multiple components of the fixed-dose compositions are made in single dose packets (sachets). For this purpose all ingredients are homogeneously blended and packed in single dose packets, also known as sachets. All the compositions according to embodiments (1) to (10) can be assembled in the form of sachets or packets.
   The packets or sachets may be used, that is administered equally to the tablets. They can be administered separately according to the medical prescriptions of the practitioner or in sequential therapy programs as described above.
(12) Furthermore, all the embodiments described above can be made in the form of pre-mix powders to be constituted with specified volume of purified water to make, e.g. 100 ml syrup prior to use. For this purpose all ingredients according to one of embodiments (1) to (10) are homogeneously blended and packed in suitable containers, preferably in 100 ml semi-opaque plastic or amber glass bottles.
   Prior to administration the pre-mix ingredients in the bottle are reconstituted with specified volume of water, preferably degassed purified water (supplied e.g. in separate plastic sachet) and administered as described above with respect to the single embodiments in detail.
(13) The pre-mix powders as described in embodiment (12) may also be formulated as effervescent packets. The engredients of the embodiments for the compositions as described are homogeneously blended in combination with specified amount of effervescent salt, preferably composed from mixture of sodium bicarbonate and anhydrous citric acid, and packed in single dose packets. The packets are administered after being effervesces in about between 50 to 100 ml of pure water.
(14) According to yet further embodiments biphasic tablets are composed from enteric coated cores housed in instantaneous release outer compressed tablets. The core tablets contain in any case the specified amount of PPI, preferably plus part of the antibiotic or combination of antibiotics. The outer immediate release housing tablets contain the remainder of antibiotic with or withour taurolidine. The taurolidine may be replaced with the zinc amino acid chelate, or both the taurolidine and the zinc amino acid chelate may be present.
   A first embodiment of this biphasic type includes omeprazole plus one third of the amount of amoxicillin. The outer immediate release housing contains the remainder of amoxicillin with or without taurolidine. Tromethamole and excipients as far as suitable might be included as usual and described before.
   A second embodiment contains all the ingredients of the first biphasic embodiment but the amoxicillin is replaced by tinidazole plus clarithromycin. Preferably the doses are 250 mg tinidazole plus 200 mg of clarithromycin.
   The biphasic compositions can be used as described above in sequential therapy program or separately according to the prescription of the medical practitioner.

The foregoing descriptions illustrate the principles, preferred embodiments and methods. However, the invention should not be construed as being limited to these particular embodiments as described above. These should be regarded as illustrative rather than restrictive. The practitioner skilled in the art will be able to find more embodiments and examples without departing from the scope of the invention as defined in the claims.

The multiple unit forms including the compressed tablets, biphasic tablets, packets, premix syrups are all designed to produce immediate therapeutic effect as they release their active ingredients in the stomach rather than other compositions of the state of the art. With other enteric coated products it takes longer time to exert their effects as they release their active ingredients in the duodenum. The advantage of the said biphasic core and shell tablets is the instantaneous release of the majority of their antibiotic contents from the housing tablet then release the protected PPI from the enteric coated core together with part of the protected antibiotics, the way by which maintain constant therapeutic effect of the antibiotics.

According to one aspect of the invention the preparations in the presented embodiments are suitably administered in sequential way. The treatment starts by administering the composition the includes amoxicillin, PPI with/without the taurolidine twice daily for five consecutive days, followed by administering the composition that includes clarithromycin, tinidazole and the PPI together with/without the taurolidine twice daily for another five consecutive days. The physician may depart from this regimen depending from the individual necessities.

The preparations in the form of premix syrups and/or dispersible packets are especially suitable for the treatment of children, elderly and patients with swallowing problems. The dosage forms is to be dispersed in pure, preferably purified water before being orally administered or fed through a naso-gastric tube.

The two general types of the pharmaceutical compositions of the invention, that is, the dosage forms a) instant release formulation and b) biphasic release form, will now be described by way of example with reference to the accompanying drawings, in which
Figure 1 is a schematic cross-section of the monophasic instant release tablet; and
Figure 2 is a schematic cross-section of the biphasic release tablet.

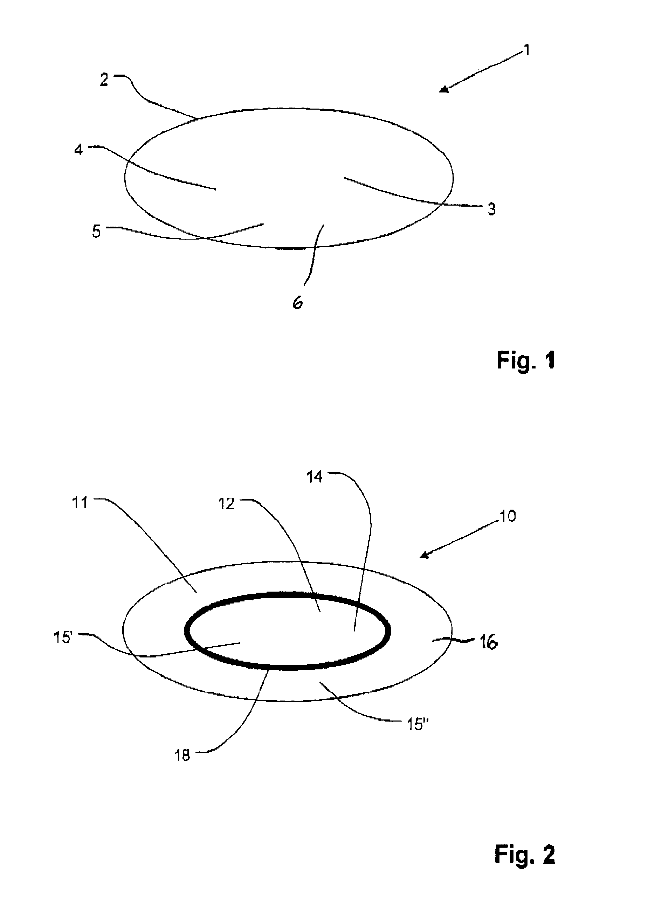

Figure 1 shows a schematic cross-section of instant release tablet 1. The compressed tablet 1 is film coated with a film coating 2 for an easier swallowing. The tablet 1 comprises only one layer 3 which contains the active ingredients. These comprise i.a. the PPI and at least one antibiotic, here are shown omeprazole 4 in a dose from 20 to 40 mg and amoxicillin 5 in a dose from 500 to 1000 mg. After administration the active ingredients 4, 5 are immediately released in the stomach. To protect omeprazole 3 from the stomach acid the layer 3 also contains tromethamole 6 which acts as a buffering alkalinizer. The tablet 1 also comprises pharmaceutically acceptable excipients as described above and in the examples below.

Figure 2 is a schematic cross-section of the biphasic release tablet 10. The tablet 10 consists of two layers, an inner, slow release core 12 and an outer, fast release layer 11. Both layers 11, 12 are separated by an enteric coating 18. The inner core contains omeprazole 14 in a dose of 20 mg and amoxicillin 15' in a dose of 333.3 mg. The outer layer contains amoxicillin 15" in a dose of 666.6 mg, so the biphasic release tablet 10 contains an amount of 1000 mg amoxicillin 15 totally. Other ingredients are contained as outlined in the embodiments and the examples (here at least tromethamole 16). Both layers 11, 12 comprise pharmaceutically acceptable excipients (as described above and in the examples below).

After administration the outer layer 11 disintegrates in the stomach and two third of the amoxicillin 15" is immediately released. Therefore, it has an immediate antibiotic effect. However the inner core 12 remains stable. Only when the inner core 12 has reached the small intestine it disintegrates and the omeprazole 14 and the remaining amount of the amoxicillin 15 is released. Because of the sustained release of the amoxicillin 15" it has a prolonged antibiotic effect. Further ingredients are present in accordance with the different embodiments according to the invention as described above and in the examples below.

The biphasic release tablet 10 has the advantage that it is formulated as a fixed dose combination which provides a better patient compliance. Another benefit is the biphasic release of the antibiotic.

The invention is now further illustrated in more detail by the way of the following examples.

### EXAMPLES for compositions

Examples 1 and 2: Instant release, multiple components compressed tablets comprising omeprazole, stabilized by tromethamole, and antibiotic (batch size 1,000 tablets)

| Ingredients | Ex. 1 - g/1000 Tablets; mg/tablet | Ex. 2 - g/1000 tablets; mg/tablet |
|---|---|---|
| PPI (omeprazole) | 20 | 20 |
| Amoxicillin | 1000 | --- |
| Clarithromycin | --- | 400 |
| Tinidazole | --- | 500 |
| Tromethamole | 150 | 150 |
| Starch glycolate | 50 | 50 |
| Aerosil® RTM | 4 | 4 |
| Sodium lauryl sulphate | 3.2 | 3.2 |
| Microcrystalline cellulose | 150 | 150 |
| Povidone K90 | 50 | 50 |
| Sodium stearyl fumarate | 18 | 18 |
| Purified water | 450 | 450 |

For preparation sodium lauryl sulphate and povidone K90 are dissolved in purified water to form the granulation liquid. Magnesium omeprazole, antibiotic (amoxicillin or clarithromycin plus tinidazole), tromethamole, microcrystalline cellulose, sodium starch glycolate and Aerosil.RTM are dry-mixed. The granulation liquid is added to the powder mixture and the mass is wet-mixed. The wet mass is dried in a steam-oven. The prepared granulation is milled through sieve 1 mm in oscillating mill equipment. The prepared granules and sodium stearyl fumarate are mixed and compressed into tablets using a rotary tableting machine equipped with 8.5 x 19 mm oval punches. The prepared tablets are film coated.

Examples 3 and 4: Instant release, multiple components compressed tablets comprising omeprazole, stabilized by tromethamole, antibiotic and taurolidine(batch size 1,000 tablets)

| Ingredients | Ex. 3 - g/1000 Tablets; mg/tablet | Ex. 4 - g/1000 tablets; mg/tablet |
|---|---|---|
| PPI (omeprazole) | 20 | 20 |
| Amoxicillin | 1000 | --- |
| Clarithromycin | --- | 400 |
| Tinidazole | --- | 500 |
| Taurolidine in microcrystalline form | 300 | 300 |
| Tromethamole | 150 | 150 |
| Starch glycolate | 50 | 50 |
| Aerosil® RTM | 4 | 4 |
| Sodium lauryl sulphate | 3.2 | 3.2 |
| Microcrystalline cellulose | 150 | 150 |
| Povidone K90 | 50 | 50 |
| Sodium stearyl fumarate | 18 | 18 |
| Purified water | 450 | 450 |

Preparation as for examples 1 and 2; the taurolidine is added to the powder mixture which is wet-mixed with the granulation liquid afterwards.

Examples 5 and 6: Instant release, multiple components compressed tablets comprising omeprazole, stabilized by tromethamole, antibiotic and Zn-amino acid chelate (batch size 1,000 tablets)

| Ingredients | Ex. 5 - g/1000 Tablets; mg/tablet | Ex. 6 - g/1000 tablets; mg/tablet |
|---|---|---|
| PPI (omeprazole) | 20 | 20 |
| Amoxicillin | 1000 | --- |
| Clarithromycin | --- | 400 |
| Tinidazole | --- | 500 |
| Zinc amino acid chelate | 75 | 75 |
| Tromethamole | 150 | 150 |
| Starch glycolate | 50 | 50 |
| Aerosil® RTM | 4 | 4 |
| Sodium lauryl sulphate | 3.2 | 3.2 |
| Microcrystalline cellulose | 150 | 150 |
| Povidone K90 | 50 | 50 |
| Sodium stearyl fumarate | 18 | 18 |
| Purified water | 450 | 450 |

Preparation as above; the zinc amino acid chelate is in the dry mixture for granulation.

Examples 7 - 10: The following examples are related to instant release, multiple components compressed tablets comprising omeprazole as the PPI, stabilized by tromethamole, various antibiotics or antibiotic mixtures, and both taurolidine and Zn-amino acid chelate (batch size 1,000 tablets), the preparation is the same as previously described

| Ingredients | Ex. 7 9/1000 tablets; mg/tablet | Ex. 8 g/1000 tablets; mg/tablet | Ex. 9 g/1000 tablets; mg/tablet | Ex. 10 g/1000 tablets; mg/tablet |
|---|---|---|---|---|
| PPI (omeprazole) | 20-40 | 20-40 | 20-40 | 20-40 |
| Amoxicillin | 1000 | --- | 1000 | --- |
| Clarithromycin | --- | 400 | --- | 400 |
| Tinidazole | --- | 500 | --- | 500 |
| levofloxacin | 250 | 250 | --- | --- |
| taurolidine | 100 | 100 | 100 | 100 |
| Zinc amino acid chelate | 75 | 75 | 75 | 75 |
| Tromethamole | 150 | 150 | 150 | 150 |
| Starch glycolate | 50 | 50 | 50 | 50 |
| Aerosil® RTM | 4 | 4 | 4 | 4 |
| Sodium lauryl sulphate | 3.2 | 3.2 | 3.2 | 3.2 |
| Microcrystalline cellulose | 75 | 75 | 75 | 75 |
| Povidone k90 | 25 | 25 | 25 | 25 |
| Sodium stearyl fumarate | 18 | 18 | 18 | 18 |
| Purified water | 450 | 450 | 450 | 450 |

### Examples 11 - 13: Stabilized, dispersible, multiple components powder/granules in packets (batch size 1,000 packets)

| Ingredients | Ex.11 | Ex. 12 | Ex. 13 |
|---|---|---|---|
| PPI (omeprazole) | 20 | 20 | 20 |
| Amoxicillin | 1000 | 1000 | 1000 |
| Tromethamole | 150 | 150 | 150 |
| Taurolidine | --- | 300 | --- |
| Zinc amino acid chelate | --- | --- | 75 |
| Xanthan gum | 35 | 35 | 35 |
| Carboxymethylcellulose | 35 | 35 | 35 |
| Saccharine Ph. Eur.3 ccrystalline | 30 | 30 | 30 |
| Pro sweet | 30 | 30 | 30 |
| Sodium lauryl sulphate | 50 | 50 | 50 |
| Sodium stearyl fumarate | 32 | 3.2 | 3.2 |
| Menthol crystals | 10 | 10 | 10 |
| Artificial Cherry flavor | Q.S. | Q.S. | Q.S. |

For further examples amoxicillin may be replaced by a combination of 400 mg clarithromycin per packet plus 500 mg of tinidazole per packet in the above examples.

For preparation 1) Magnesium omeprazole, antibiotic(s), optionally taurolidine, optionally zinc amino acid chelate, and tromethamole are dry-mixed. 2) Carboxymethylcellulose, saccharine (other artificial or natural sweeteners of approved pharmaceutical application can be used) and Pro sweet are dry mixed. 3) Xanthan gum, sodium lauryl sulphate, cherry flavour and menthol are dry mixed. 4) The mixed powders from steps 1, 2 and 3 are homogeneously blended together. 5) The homogeneous powder mixture from step 4 is packed in single dose packets in controlled humidity environment under vacuum. 6) On the other hand, the homogeneous powder from step 4 is moistened with purified water to obtain fluffy granulations, The fluffy granulation is dried in a steam oven at 50 °C, milled through sieve 1 mm in an oscillating mill and packed in single dose packets in controlled equipment and packed in single dose packets in controlled humidity environment under vacuum.

For examples of stabilized, effervescent, multiple components powder in packets the above examples 11 to 13 - or further powder examples in accordance with the embodiments discussed above - are complemented with Q.S. of effervescent salt.

Examples 14 - 17: biphasic enteric core tablets comprising mixture of omeprazole plus antibiotics housed within immediate release compressed coat of antibiotic plus taurolidine and/or zinc amino acid chelate (batch size 1,000 tablets):

| Ingredients | Ex. 14 g/1000 tablets; mg/tablet | Ex. 15 g/1000 tablets; mg/tablet | Ex.16 g/1000 tablets; mg/tablet | Ex. 17 g/1000 tablets; mg/tablet |
|---|---|---|---|---|
| PPI (omeprazole) | 20 | 20 | 20 | 20 |
| Amoxicillin | 1000 | 1000 | --- | --- |
| Clarithromycin | --- | --- | 400 | 400 |
| Tinidazole | --- | --- | 500 | 500 |
| Tromethamole | 150 | 150 | 150 | 150 |
| Taurolidine | 300 | --- | 300 | --- |
| Zinc amino acid chelate | --- | 75 | --- | 75 |
| Starch glycolate | 50 | 50 | 50 | 50 |
| Aerosil® RTM | 4 | 4 | 4 | 4 |
| Sodium lauryl sulfat | 3.2 | 3.2 | 3.2 | 3.2 |
| Microcrystalline cellulose | 150 | 150 | 150 | 150 |
| Povidone K90 | 50 | 50 | 50 | 50 |
| Sodium stearyl fumarate | 18 | 18 | 18 | 18 |
| Purified water | 450 | 450 | 450 | 450 |
| Solution for separating layer (for 10 kg tablets): | | | | |
| Hydroxypropylmethylcellulose | 300 g | 300 g | 300 g | 300 g |
| Hydrogen peroxide (30%) | 0 003 g | 0.003 g | 0 003 g | 0.003 g |
| Purified water | 2701 g | 2701 g | 2701 g | 2701 g |
| Solution for enteric coat ing layer (for 10 kg tablets): | | | | |
| Methacrylic acid copolymer | 2501 g dispersion (30%) | 2501 g dispersion (30%) | 2501 g dispersion (30%) | 2501 g dispersion (30%) |
| Polyethylene glycol 400 | 81g | 81 g | 81 g | 81 g |
| Titanium dioxide | 101 g | 101 g | 101 g | 101 g |
| Purified water | 1961 g | 1961 g | 1961 g | 1961 g |

In examples 14 to 17 the tromethamole may be left out (examples 18-21)

For preparation sodium lauryl sulphate and povidone K90 are dissolved in purified water to form the granulation liquid. Omeprazole plus about one third of amoxicillin or about one third of the clarithromycin tinidazole mixture plus one third of each of microcrystalline cellulose, sodium starch glycolate and Aerosil ® RTM are dry-mixed. Specified amount of the granulating liquid is added to the powder mixture and the mass is wet-mixed. The wet mass is dried in a steam-oven. The prepared granulation is milled through sieve 1 mm in an oscillating granulator. The prepared granules and one third of sodium stearyl fumarate are mixed and compressed into tablets using a rotary tableting machine equipped with a suitable size biconvex or oval punch. The obtained core tablets are covered with a separating layer and an enteric tablet cating layer containing suitable plasticizer to avoid cracking of the enteric coating during housing compression inside the immediate release layer. The immediate release housing tablet is prepared by dry mixing the remainder amount of antibiotic plus taurolidine and/or zinc amino acid chelate, optionally plus tromethamole, plus the remainder of each of microcrystalline cellulose, sodium starch glycolate and Aerosil. The remainder of the granulating liquid is added to the powder mixture and the mass is wet-mixed. The wet mass is dried in oven. The prepared granulation is milled through sieve 1 mm in an oscillating granulator. The prepared granules are mixed with the remainder of sodium stearyl fumarate and compressed around the core tablets by bicota tableting machine. The amounts of ingredients can be taken from the table.

### TESTS

To further illustrate the benefits of the invention the following test results are reported:
The effect of the compositions according to the invention shall be proved. Different tumor cell types, namely gastric cancer (AGS, MKN45, and SNU16) cells were incubated with different components of the composition as well as a combination according to the finished product for 6 h. Whole-cell extracts were prepared and analyzed by Western blot analysis using antibody against CXCR4. The same blots were stripped and reprobed with β-actin antibody to show equal protein loading. Representative results of three independent experiments are shown.

The same experiment was carried out to find out whether the composition downregulates expression of CXCR4 in gastric (AGS, MKN45, and SNU16) cancer cell lines, which has never been investigated before. Cells were treated with 5 µm active ingredients of Ex. 6 and its major active components for 6 h before assessing the resultant effect on CXCR4 expression. This clearly demonstrates that the composition substantially downregulated CXCR4 expression in all three gastric cancer cell lines. Result showed convincingly that CXCR4 downregulation by the composition is cell type-specific.

| | Fold change | | | |
|---|---|---|---|---|
| Concentration µm | Finished product | PPI | CLR | TND |
| 0 | 1.0 ± 0.01 | 1.1 ± 0.03 | 1.1 ± 0.01 | 1.0 ± 0.02 |
| 0.5 | 1.1 ± 0.02 | 1.4 ± 0.02 | 1.3 ± 0.01 | 1.4 ± 0.01 |
| 1.0 | 0.94 ± 0.01 | 0.96 ± 0.03 | 0.97 ± 0.01 | 0.98 ± 0.01 |
| 2.5 | 0.78 ± 0.02 | 0.81 ± 0.05 | 0.79 ± 0.04 | 0.85 ± 0.01 |
| 5.0 | 0.42 ± 0.01 | 0.46 ± 0.02 | 0.44 ± 0.01 | 0.53 ± 0.03 |

| | | | | |
|---|---|---|---|---|
| PPI = proton pump inhibitor (Omeprazol) CLR = clarithromycin TND = tinidazole | | | | |

The composition suppresses migration of gastric cancer cells. The wound-healing assay serves for evaluating the inhibitory effect of the composition on MDA-MB-231 cell migration. Confluent monolayers of MDA-MS-231 cells were scarred, and repair was monitored microscopically after 6 h of pre-treatment with 5 µm of the composition according to Ex. 6 and its major components before being exposed to 100 ng/ml CXCL12 for 24 h. Width of wound was measured at time zero and 24 h of incubation with and without the active ingredients of the composition and its components in the absence or presence of CXCL12 in RPMI medium containing 1 % serum. The representative investigations showed the same area of wounds at time zero and after 48 h of incubation.

| | | **mRNA fold change** | | |
|---|---|---|---|---|
| Time (hours) | **Finished product** | **PPI** | **CLR** | **TND** |
| 0 | 0.84 ± 0.01 | 0.92 ± 0.02 | 0.90 ± 0.05 | 0.94 ± 0.02 |
| 2 | 0.72 ± 0.05 | 0.88 ± 0.021 | 0.78 ± 0.02 | 0.83 ± 0.01 |
| 6 | 0.50 ± 0.02 | 0.67 ± 0.06 | 0.63 ± 0.03 | 0.79 ± 0.04 |
| 8 | 0.38 ± 0.02 | 0.54 ± 0.04 | 0.50 ± 0.03 | 0.65 ± 0.03 |
| 12 | 0.21 ± 0.02 | 0.41 ± 0.04 | 0.42 ± 0.03 | 0.51 ± 0.03 |
| 24 | 0.08 ± 0.02 | 0.38 ± 0.04 | 0.37 ± 0.03 | 0.39 ± 0.03 |

The composition and its major active components suppresse invasion in breast and gastric cancer cells. (A) MDA-MB-231 (2 x 10⁵ cells) were seeded in the top-chamber of the Matrigel. After pre-incubation with or without the composition according to Ex. 6 and its major active components (5 µm) for 6 h, transwell chambers were then placed into the wells of a 24-well plate, in which we had added either the basal medium only or basal medium containing 10 ng/ml GXCL12 for 24 h. After incubation, they were assessed for cell invasion. Columns indicate mean percentage of invaded cells; bars, S.E. *, P > 0.05. (B) AGS (2 x 10⁵ cells) were seeded in the top-chamber of the Matrigel. After pre-incubation with or without the composition and its major active components (5 µm) for 6 h, transwell chambers were then placed into the wells of a 24-well plate, in which we had added either the basal medium only or basal medium containing 100 ng/ml CXCL12 for 24 h. After incubation, the chambers were assessed for cell invasion. Columns indicate mean percentage of invaded cells; bars, S.E. *, P > 0.05. Representative results of three independent experiments are shown. (C) The composition and its major active components suppresses expression of CXCR4 mRNA expression in gastric cancer cells. AGS cells were treated with 5 µm of the composition and its major active components for indicated times. Total RNA was isolated and analyzed by RT-PCR assay. 18S was shown to equal loading of total RNA. Representative results of three independent experiments are shown. (D) The composition and its major active components suppresses expression of CXCR4 protein expression in gastric cancer cells. Cells were incubated with 5 µm of the active ingredients of the composition and its single major active components for indicated times. Whole-cell extracts were prepared and analyzed by Western blot analysis using antibodies against CXCR4. The same blots were stripped and reprobed with ß-actin antibody to show equal protein loading. Representative results of three independent experiments are shown.

| | | **Percentage of invade cells** | | |
|---|---|---|---|---|
| Time (hours) | **Finished product** | **PPI** | **CLR** | **TND** |
| **0** | **85.23 ± 11.51** | **85.23 ± 11.51** | **85.23 ± 11.51** | **85.23 ± 11.51** |
| **2** | **78.15 ± 12.22** | **82.10 ± 15.10** | **80.22 ± 10.25** | **81.10 ± 14.20** |
| **6** | **57.57 ± 7.15** | **74.11 ± 6.25** | **77.26 ± 9.14** | **72.55 ± 8.10** |
| **8** | **44.63 ± 4.55** | **60.12 ± 11.50** | **62.27 ± 6.50** | **64.65 ± 10.11** |
| 12 | **31.25 ± 6,35** | **49.25 ± 7.26** | **55.20 ± 7.10** | **51.33 ± 8.20** |
| **24** | **11.22 ± 2.11** | **39.25 ± 4.15** | **48.20 ± 6.15** | **42.25 ± 8.55** |

## Claims

1. An oral pharmaceutical composition for treatment of helicobacter pylori associated diseases comprising
- a proton pump inhibitor,
- an antibiotic or a combination of antibiotics,
- additionally at least one agent selected from the group consisting of taurolidine or taurultam or a combination thereof, a zinc amino acid chelate, tromethamole,
within a fixed-dose combination for oral administration in the form of either an instant release formulation or a biphasic release form, wherein the tromethamole is always present in the instant release formulations.

2. A pharmaceutical composition according to claim 1, wherein the antibiotic is amoxicillin or a combination of tinidazole and clarithromycin.

3. A pharmaceutical composition according to claim 1 or 2, wherein the proton pump inhibitor is omeprazole.

4. A pharmaceutical composition according to one of the preceding claims, wherein it is formulated as capsules, effervescent granules or powder, effervescent tablets, liquids, dispersible and/or soluble tablets, dispersible powder, suspensions, sachets, premix syrups, jellies, glycerites or tablets.

5. A pharmaceutical composition according to claim 4, wherein the tablets are compressed tablets or biphasic tablets with an inner slow release layer and an outer fast release layer, preferably wherein the outer layer is formulated in a compressed form.

6. A pharmaceutical composition according to one of claims 1 to 5 in a biphasic release form, wherein the inner layer or slow release phase composition is enteric coated and comprises the proton pump inhibitor.

7. A pharmaceutical composition according to claim 6, wherein the outer layer or immediate release phase composition comprises the antibiotic or mixture of antibiotics.

8. A pharmaceutical composition according to claim 6 or 7, wherein the antibiotic or the combination of antibiotics is comprised in both of the phases, in particular both of the layers.

9. A pharmaceutical composition according to one of claims 6 to 8, wherein the substance or substances selected from taurolidine or taurultam or a combination thereof, the zinc amino acid chelate, the tromethamole is/are comprised in the outer layer of fast release phase.

10. A pharmaceutical composition according to one of claims 6 to 9, wherein the biphasic release form is a biphasic compressed tablet with an enteric coating on the core phase comprising a pharmaceutically suitable plasticizer.

11. A method for treatment of helicobacter pylori associated diseases comprising administering orally to a patient in need thereof
- a proton pump inhibitor,
- an antibiotic or a combination of antibiotics,
- additionally at least one agent selected from the group consisting of taurolidine or taurultam or a combination thereof, a zinc amino acid chelate, tromethamole,
where said components are arranged within one or more pharmaceutical compositions administered concomitantly or sequentially over a time period from minutes to hours.
